# EUROPEAN PATENT SPECIFICATION

(11) **EP 4 498 075 B1**
(45) Date of publication and mention of the grant of the patent: **09.09.2026**
(21) Application number: 23774901.5
(22) Date of filing: 20.03.2023
(51) Int. Cl.: G01N 23/2252, G01N 33/20

(54) **STEEL EVALUATION METHOD AND PROGRAM**
STAHLBEURTEILUNGSVERFAHREN UND -PROGRAMM
PROCÉDÉ ET PROGRAMME D'ÉVALUATION D'ACIER

(30) Priority: 22.03.2022 JP 2022046049; 21.07.2022 WO PCT/JP2022/028367
(43) Date of publication of application: 29.01.2025
(73) Proprietor: Proterial, Ltd., Tokyo 135-0061 (JP)
(72) Inventor: FUKUMOTO, Shiho, Tokyo 135-0061 (JP)
(74) Representative: Cabinet Beaumont
(86) International application number: PCT/JP2023/011002
(87) International publication number: WO 2023/182306

(56) References cited:
- WO-A1-2021/033407
- CN-A- 112 129 755
- JP-A- 2015 178 643
- JP-A- 2020 194 691
- KR-B1- 101 615 158
- US-A1- 2021 047 705
- SANTOFIMIA M J ET AL: "Experimental study of the distribution of alloying elements after the formation of epitaxial ferrite upon cooling in a low-carbon steel", MATERIALS CHARACTERIZATION, ELSEVIER, NEW YORK, NY, US, vol. 61, no. 10, 1 October 2010 (2010-10-01), pages 937 - 942, XP027189546, ISSN: 1044-5803, [retrieved on 20100617]
- JOHN J. FRIEL ET AL: "Tutorial Review: X-ray Mapping in Electron-Beam Instruments", MICROSCOPY AND MICROANALYSIS, vol. 12, no. 01, 1 February 2006 (2006-02-01), pages 2 - 25, XP055513447, ISSN: 1431-9276, DOI: 10.1017/S1431927606060211
- MEGURO, SUSUMU: "Current Advances in Materials and Processes", ZAIRYO TO PUROSESU = CURRENT ADVANCES IN MATERIALS AND PROCESSES : CAMP-ISIJ, NIHON TEKKO KYOKAI, JP, vol. 26, no. 1, 1 March 2013 (2013-03-01), JP , pages 262, XP009550111, ISSN: 1882-8922
- SHIMIZU, TAKAYUK I, INOUE, KOICHIRO: "Influence of Coarse Carbide Volume on Anisotropy of Dimensional Change at Heat Treatment of Cold Work Die Steels", ELECTRIC STEELMAKING [DENKI-SEIKO]., vol. 78, no. 4, November 2007 (2007-11-01), pages 289 - 298, XP009110745
- ISHIDA, K. ET AL.: "Calculation of the effect of alloying elements on the Ms temperature in steels", JOURNAL OF ALLOYS AND COMPOUNDS, vol. 220, 1 April 1995 (1995-04-01), pages 126 - 131, XP004066390, DOI: 10.1016/0925-8388(94)06002-9

## Description

### Field of the invention

The present invention relates to a steel evaluation method and a program.

### Background of the invention

Steels used in bending, drawing, and punching of plate materials are generally manufactured by performing annealing, cutting, quenching, and tempering treatment. As a problem that occurs during a quenching or tempering process, a change in dimensions (dimensional change) is known, and when the dimensional change is large, this leads to an increase in the number of processing steps after the heat treatment, and thus various examinations have been made for suppressing the dimensional change in the related art.

For example, in Patent Literature 1, the applicant of this application has found that a heat-treatment dimensional change can also be suppressed by reducing segregation in order to reduce the heat-treatment dimensional change occurring after quenching and tempering, and has suggested a cold die steel capable of suppressing the dimensional change by employing a "segregation index K" in which a concentration distribution rate of solid and liquid phases of each element and a variation in specific gravity are combined and by optimally adjusting the value to reduce the segregation, thereby suppressing the dimensional change.

In addition, in order to suppress the dimensional change, Patent Literature 2 discloses a cold die steel in which a primary carbide is reduced, to which an appropriate amount of Ni, Al, and Cu is added on the basis of a component composition capable of suppressing occurrence of the dimensional change as much as possible within a range satisfying various characteristics, and which is excellent in dimensional change control characteristics. According to the invention described in Patent Literature 1, Ni and Al form an intermetallic compound, precipitate during tempering (aging) in a secondary hardening region of a tool steel, and act on dimensional change in a contraction direction, and thus it is possible to offset expansion during a heat treatment.

### Citation List

### Patent Literature:

Patent Literature 1: Japanese Patent Laid-Open Publication No. 2006-152356
Patent Literature 2: Japanese Patent Laid-Open Publication No. 2006-169624

### Non Patent Literature:

Non Patent Literature 1: G. A. Roberts, Tool Steel 5th (1998) p 338
Non Patent Literature 2: F. Rapatz, Die Edelstahl (1962), p 945
Non Patent Literature 3: "Influence of Coarse Carbide Volume on Anisotropy of Dimensional Change at Heat Treatment of Cold Work Die steels", Takayuki Shimizu and Koichiro Inoue, Electric Steelmaking, 2007, 78.4: 289
Non Patent Literature 4: Santofimia et al, "Experimental study of the distribution of alloying elements after the formation of epitaxial ferrite upon cooling in a low-carbon steel", Materials Characterization, vol. 61, no. 10 (2010), p.937-342

### Summary

### Problems to be Solved by Invention

On the other hand, according to experimental results obtained by the present applicant, there is a case where the dimensional change characteristics vary even in the same alloy composition. In a case where a shape of a mold is rectangular parallelepiped, the variation is expressed as anisotropy when a rate of change in thickness, width, and length after quenching and tempering is different as compared with dimensions before quenching. So far, as metallurgical technical literatures on the anisotropy of the dimensional change, there have been two report examples (Non Patent Literatures 1 and 2) for an observed dimensional change shape. According to a study example on cold tool steel (cold die steel), if there are no crystallized carbides in the cold die steel, the dimensional change is less likely to occur, and there is a report (Non Patent Literature 3) that anisotropy occurs when crystallized carbides exist on the basis of FEM analysis considering a distribution of the crystallized carbides, and thus quality stabilization when being evaluated by the dimensional change is also a problem. In addition, as is also disclosed in Patent Literature 1, the dimensional change can be improved by eliminating component segregation, but the degree of influence on the dimensional change is different for each element, and time is taken to know which element species should be focused to improve the segregation.

Non patent literature 4 suggests the use of electron probe microanalysis (EPMA) for mapping phases in steel samples, specifically martensite/retained austenite, epitaxial ferrite and intercritical ferrite.

Here, an object of the invention is to provide an evaluation method capable of obtaining steel with stable quality by using a new index for evaluating a dimensional change.

### Means for Solving Problems

Specifically, according to an aspect of the invention, there is provided a steel evaluation method including: a process of measuring a surface of steel with an electron probe micro analyzer to obtain mapping data of a base composition; a process of obtaining mapping data of an Ms temperature from the mapping data of the base composition; a process of binarizing the mapping data of the Ms temperature; a process of obtaining a first main component and a second main component by main component analysis of the mapping data of the Ms temperature which has been subjected to the binarization processing; and a process of evaluating dimensional change characteristics of the steel on the basis of a relationship between the first main component and the second main component.

### Effects of Invention

According to the disclosure, it is possible to provide an evaluation method for obtaining steel with stable quality.

### Brief Description of Drawings

FIG. 1 is an Ms-temperature mapping diagram.
FIG. 2 is a view after binarization of FIG. 1.
FIG. 3 is a view in which a main component vector obtained by main component analysis is superimposed on FIG. 1.
FIG. 4 is a schematic view illustrating an EPMA measurement site in examples.
FIG. 5 is a view illustrating main component analysis results in a T-L direction of Sample No. 1.
FIG. 6 is a view illustrating main component analysis results in a T-W direction of Sample No. 1.
FIG. 7 is a view illustrating main component analysis results in a W-L direction of Sample No. 1.
FIG. 8 is a view illustrating main component analysis results in the T-L direction of Sample No. 2.
FIG. 9 is a view illustrating main component analysis results in the T-W direction of Sample No. 2.
FIG. 10 is a view illustrating main component analysis results in the W-L direction of Sample No. 2.
FIG. 11 is a graph of actually measured heat-treatment dimensional change.
FIG. 12 is a view illustrating main component analysis results in the T-L direction of Sample No. 3.
FIG. 13 is a view illustrating main component analysis results in the T-L direction of Sample No. 4.
FIG. 14 is a view illustrating main component analysis results in the T-L direction of Sample No. 5.
FIG. 15 is a view illustrating main component analysis results in the T-L direction of Sample No. 6.
FIG. 16 is a graph of actually measured heat-treatment dimensional change of Sample No. 3.
FIG. 17 is a graph of actually measured heat-treatment dimensional change of Sample No. 4.
FIG. 18 is a graph of actually measured heat-treatment dimensional change of Sample No. 5.
FIG. 19 is a graph of actually measured heat-treatment dimensional change of Sample No. 6.
FIG. 20 is an Ms-temperature mapping image of Sample No. 7B.
FIG. 21 is a view illustrating main component analysis results of Sample No. 7B.
FIG. 22 is an Ms-temperature mapping image of Sample No. 7T.
FIG. 23 is a view illustrating main component analysis results of Sample No. 7T.
FIG. 24 is an Ms-temperature mapping image of Sample No. 12B.
FIG. 25 is a view illustrating main component analysis results of Sample No. 12B.
FIG. 26 is an Ms-temperature mapping image of Sample No. 12T.
FIG. 27 is a view illustrating main component analysis results of Sample No. 12T.
FIG. 28 is an Ms-temperature mapping image of Sample No. 14B.
FIG. 29 is a view illustrating main component analysis results of Sample No. 14B.
FIG. 30 is an Ms-temperature mapping image of Sample No. 14T.
FIG. 31 is a view illustrating main component analysis results of Sample No. 14T.
FIG. 32 is an explanatory view illustrating evaluation results of a main component.
FIG. 33 is a scatter plot of a main component of Sample No. 7.
FIG. 34 is a scatter plot of a main component of Sample No. 12.
FIG. 35 is a scatter plot of a main component of Sample No. 14.
FIG. 36 is an explanatory view illustrating a configuration of an information processing apparatus.
FIG. 37 is a flowchart illustrating a processing flow of a program.

### Description of Embodiments

First, the present inventors have conducted an investigation from the viewpoint that a dimensional change occurs due to a difference in an Ms temperature with respect to surroundings instead of the viewpoint that the dimensional change occurs due to segregation of individual elements. That is, the present inventors have made a thorough investigation on an analysis method with focus given to an Ms-temperature distribution which allows comprehensive evaluation of the content of all of main elements (for example, C, Si, Mn, and a carbide forming element) as a "single control parameter" instead of the content of each of the main elements as a parameter that has an influence on dimensional change characteristics. In addition, the present inventors obtained a finding that the dimensional change can be improved by equalizing a temperature difference between an Ms temperature at a certain measurement point and a surrounding point, and a direction of the temperature difference. As a result, they conducted main component analysis on a mapping image of the Ms temperature, found that the obtained magnitude or direction of the main component has a correlation with dimensional change characteristics, and accomplished the invention based on the gist of controlling the magnitude or direction of the main component.

Description will be made sequentially with reference to specifical examples of an evaluation method of the invention.

First, a surface of a steel material is measured by a field emission-electron probe micro analyzer (FE-EPMA) to obtain mapping data of a base composition in a measurement field of view. Specifically, a sample having dimensions of 15 mm × 20 mm × 10 mm from any site of the steel material, is mirror polished, and is observed by EPMA. With regard to measurement conditions in the FE-EPMA, for example, with respect to a measurement field of 8 mm × 4 mm, observation may be conducted under conditions in which a beam diameter is set to 20 µm, an acceleration voltage is set to 15 kV, and an irradiation current is set to 0.1 µA. Here, a mapping target is to obtain mapping data for main elements of the steel as described above.

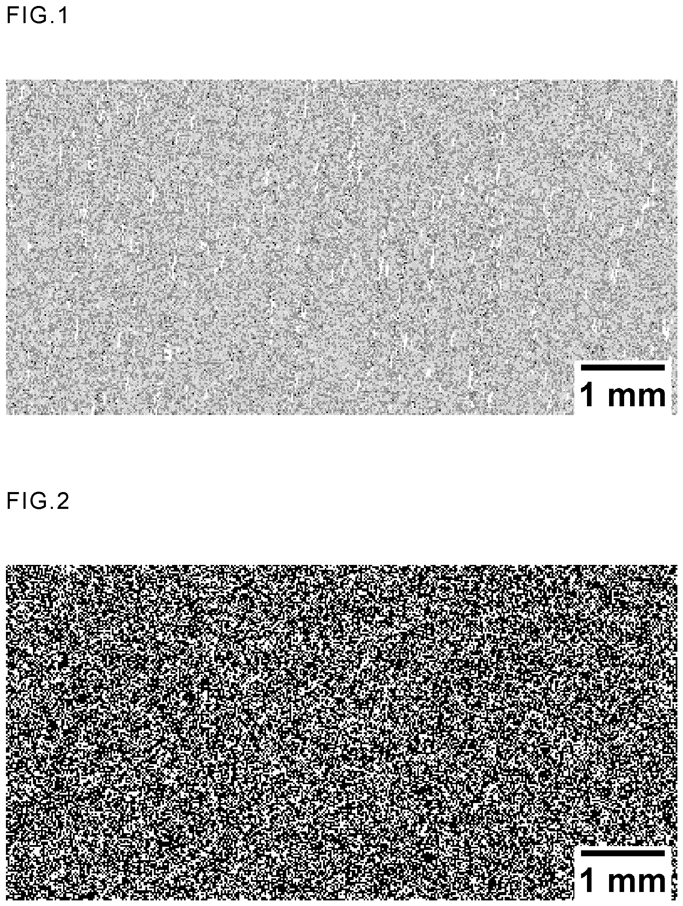

Next, mapping data of an Ms temperature as shown in FIG. 1 is obtained from the acquired mapping data. The Ms temperature is a temperature at which a crystal structure starts to transform from an fcc structure to a bcc structure without diffusion from austenite that is a crystal structure of the steel to a martensite structure, and when being cooled from a temperature higher than the temperature and being caused to pass, the crystal of the steel shows expanding characteristics. In order to acquire the above-described Ms-temperature mapping data, for example, by using a calculation method of an Ms-temperature prediction formula by Ishida (ISHIDA, K. Calculation of the effect of alloying elements on the Ms temperature in steels. Journal of Alloys and Compounds, 1995, 220. 126 to 131.), it is possible to obtain Ms-temperature mapping data (TIFF image constructed with 8 bits, 16 bits, 24 bits, 32 bits, or more bits). In addition, it is also possible to use another characteristic value other than the Ms-temperature prediction formula. Specifically, it is also possible to show anisotropy of a certain property by using a measured value of hardness of a minute portion of a base portion with a nano indenter, an experimental formula for Ac1 temperature and Ac3 temperature described in "steel material" in a known literature (for example, "Lecture on Current Metallurgy, Materials Volume 4, Steel Materials, The Japan Institute of Metals, 1985."), or a value obtained from an Ni equivalent and a Cr equivalent shown in Schaeffler phase diagram. Note that, when using the prediction formula, an Ms temperature of pure iron is set to 545°C for convenience so as to exclude a structure that does not undergo martensite transformation. At this point, a mapping image of the Ms temperature is obtained, and a height difference of the Ms temperature can be shown, but it is difficult to evaluate the density of a contour line from a contour map of the Ms temperature for each fine measurement point. Accordingly, in this embodiment, binarization processing and main component analysis to be described later are performed. Here, for example, in a measurement field of view of 8 mm × 4 mm of the EPMA, the number of measurement pixels of obtained Ms-temperature mapping may be 400 pixels (horizontal) × 200 pixels (vertical), but in this case, analysis may not converge in a unit of 1 pixel, and a high-precision result may not be obtained. In that case, when increasing the pixels by enlarging the image by an integer multiple, a high-precision result tends to be obtained, and thus this case is preferable.

FIG. 1 illustrates an example of a mapping image obtained by the above-described processing. A measurement field of view is 8 mm (horizontal) and 4 mm (vertical), and the number of measurement points of the Ms temperature is set to 400 points in a horizontal direction and 200 points in a vertical direction, and the sum of the measurement points is 80000 points. Due to limitation of electronic application software used for international patent application, an image is in black and white, but actually, the Ms temperature measured at respective measurement points is color mapped with a blue color on a low-temperature side and a red color on a high-temperature side. A white portion represents a measurement point at which the Ms temperature cannot be evaluated because martensite transformation does not occur.

Note that, in a case where an image constructed from Ms-temperature mapping data obtained by the above-described method is shown as it is, there is a possibility that a high-precision result may not be output in main component analysis to be described later, and thus, for example, it is preferable to perform normalization processing (equalization processing) from a minimum value to a maximum value before binarization processing to be described later by using commercially available image processing software or the like such as ImageJ. The normalization processing performs color-coding (subtractive color conversion) for each hierarchy according to a data structure called a look-up table which is created to improve efficiency by replacing Ms-temperature data with simple array reference processing and in which distribution of innumerable Ms temperatures are divided into equal-spaced hierarchies. As the look-up table, a look-up table equipped with commercially available image processing software may be used.

Next, binarization processing is conducted on the acquired Ms-temperature mapping image to obtain a black and white image as shown in FIG. 2. As a threshold value determination method according to the binarization processing, a discriminant analysis method (Otsu method), a mode analysis method, and the like are present, but with regard to the threshold value in the binarization processing in the disclosure, the discriminant analysis method is used. This method is a binarization method based on a threshold value with which the degree of separation expressed as a ratio of an interclass variance and an in-class variance becomes maximum, and the threshold value can be automatically set. The Otsu method can be selected as a binarization method in commercially available image processing software such as ImageJ. In addition, the Otsu method can also be designated in an image processing function of general-purpose programming language Python as cross-platform software.

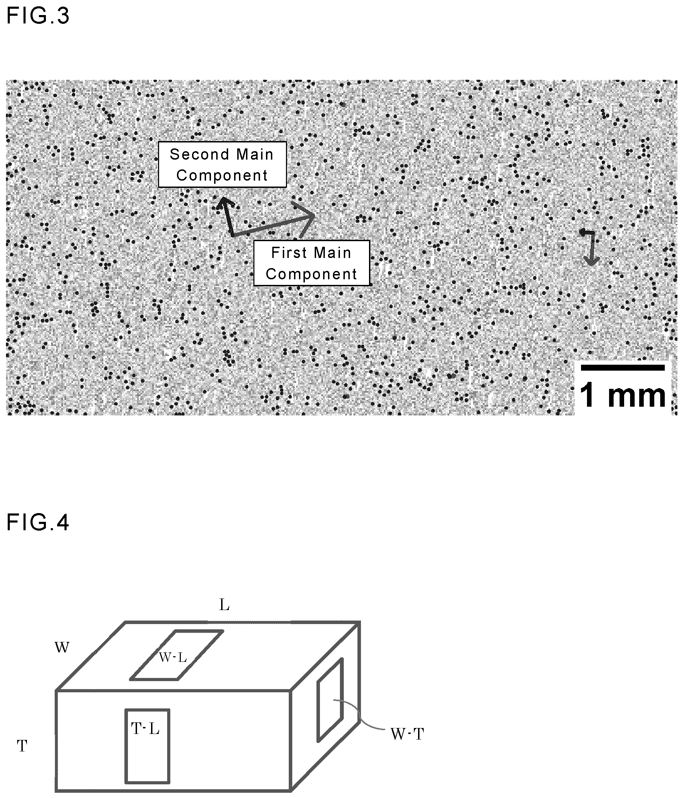

Next, main component analysis is conducted on the binarized Ms-temperature mapping image to calculate a first main component and a second main component in the Ms temperature. Through this process, a magnitude of the first main component (a main component vector with the largest eigenvalue) and a magnitude of the second main component (a main component vector with a largest eigenvalue next to the first main component) of the Ms temperature at each measurement site can be known as illustrated in FIG. 3. Note that, FIG. 3 is shown as a black and white image due to limitation of electronic application software used for international patent application, but actually, FIG. 3 is a view in which the first main component is indicated by a red arrow and the second main component is indicated by a blue arrow. In addition, the calculated magnitude of the first main component, and a ratio between the first main component and the second main component are arranged. When this first main component is small and the ratio between the first main component and the second main component is closer to 1, a temperature difference of the Ms temperature between a measurement point and a surrounding point is small, and a distribution of the Ms temperature shows isotropy, that is, there is a high possibility that the dimensional change is less likely to occur in the steel. On the other hand, the larger the first main component is and the further the ratio between the first main component and the second main component deviates from 1 (a distribution showing a bias toward the first main component and the second main component), the larger a temperature difference of the Ms temperature between the measurement point and the surrounding point, and the further an expansion phenomenon tends to be biased in a certain direction in a thickness, a width, and a length. Therefore, the distribution of the Ms temperature shows anisotropy, and the steel material can be evaluated as steel material in which heat-treatment dimensional change is likely to occur. In addition, an angle of the first main component is also an index correlated with the heat-treatment dimensional change. A vector of the first main component and a rotation angle of the Ms-temperature mapping image with respect to the horizontal axis can be derived. When the rotation angle is biased toward a certain direction, it can be said that the steel material is a steel material in which the distribution of the Ms temperature is biased and the heat-treatment dimensional change is likely to occur. In addition, as an index indicating the bias, a rotation angle of a vector of a main component and the mapping data with respect to the vertical axis and the horizontal axis of an image, a contribution rate of each main component with respect to a total sum of the obtained main components or the main sum of the first main component, the second main component, and the like or a cumulative contribution rate thereof can also be used. When a plurality of obtained rotation angles is non-uniform, it will show a bias. When the contribution rate of each main component is also non-uniform, it will show a bias. In a case of comparing lots with different quality characteristics, it is possible to quantitatively compare bias and anisotropy characteristics by using the difference in bias by comparing the derived indexes. This method is applicable to characteristic analysis of not only forged and rolled steel materials but also cast products.

In addition, from evaluation results obtained by the main component analysis, a material in which a distribution of the Ms temperature exhibits anisotropy may be improved by appropriately adjusting components or a manufacturing method so as to exhibit isotropy. In addition, even in steel ingots, it is possible to stably obtain steel with suppressed dimensional change by selectively using a portion in which the distribution of the Ms temperature is more isotropic.

The evaluation method described above can be used to evaluate and improve heat-treatment dimensional change related to various kinds of steel such as carbon steel and alloy steel. Examples of the steel for which the heat-treatment dimensional change is important include die steel such as cold die steel and hot die steel, and cutting tool steel such as high-speed tool steel and carbon tool steel. Examples

### Example 1

Steel ingots were prepared, and the ingots were subjected to hot forging, cooling, and annealing. The annealed materials were subjected to a quenching treatment by air cooling from 1020°C. Then, after the quenching treatment, the hardness of each of the steel ingots was adjusted to 51 HRC by two times of tempering treatments at 590°C after the quenching treatment. Table 1 shows component compositions (analysis values) of Sample Nos. 1 and 2.

**[Table 1]**

| (mass%) | | | | | | | | | | |
|---|---|---|---|---|---|---|---|---|---|---|
| Sample No. | C | Si | Mn | S | Ni | Cr | Mo | V | Al | Remainder |
| 1 | 0.62 | 1.35 | 0.78 | 0.0490 | 0.10 | 4.87 | 1.37 | 0.15 | 0.085 | Fe and inevitable impurities |
| 2 | 0.40 | 0.99 | 0.40 | 0.0007 | 0.05 | 5.07 | 1.25 | 0.84 | 0.029 | Fe and inevitable impurities |

Samples having a length of 30 mm, a width of 25 mm, and a thickness of 20 mm were taken from each of the obtained quenched and tempered materials, and composition mapping data in portions of T-L, T-W, and W-L shown in FIG. 4 was acquired by using FE-EPMA. Note that, in FIG. 4, an L direction indicates a direction in which the steel ingot is extended in a hot-forging process. As measurement conditions in the FE-EPMA, a beam diameter was 20 µm, an acceleration voltage was 15 kV, and an irradiation current was 0.1 µm with respect to a measurement field of view of 8 mm × 4 mm. Elements C, Si, Mn, Cr, Mo, and V which are main elements were set as a measurement target. In addition, from composition mapping data obtained by a quantitative analysis method of the FE-EPMA, Ms-temperature mapping data was acquired by using an Ms-temperature prediction formula of Ishida. An image pixel size of the Ms-temperature mapping data was 400 pixels (horizontal) × 200 pixels (vertical). Then, the image was enlarged four times in both horizontal and vertical sizes of the image by image edition software, and the number of pixels was adjusted to four times. Then, normalization processing (equalization processing of image processing software ImageJ) was performed on from a minimum value to a maximum value. The normalization processing is processing of performing color-coding (subtractive color conversion) for each hierarchy according to a data structure called a look-up table which is created to improve efficiency by replacing Ms-temperature data with simple array reference processing and in which distribution of innumerable Ms temperatures are divided into equal-spaced hierarchies. The normalization processing was performed by executing LUT: "16 color" processing of ImageJ and as a result thereof, Ms-temperature data separated into 32 levels of hierarchy was obtained.

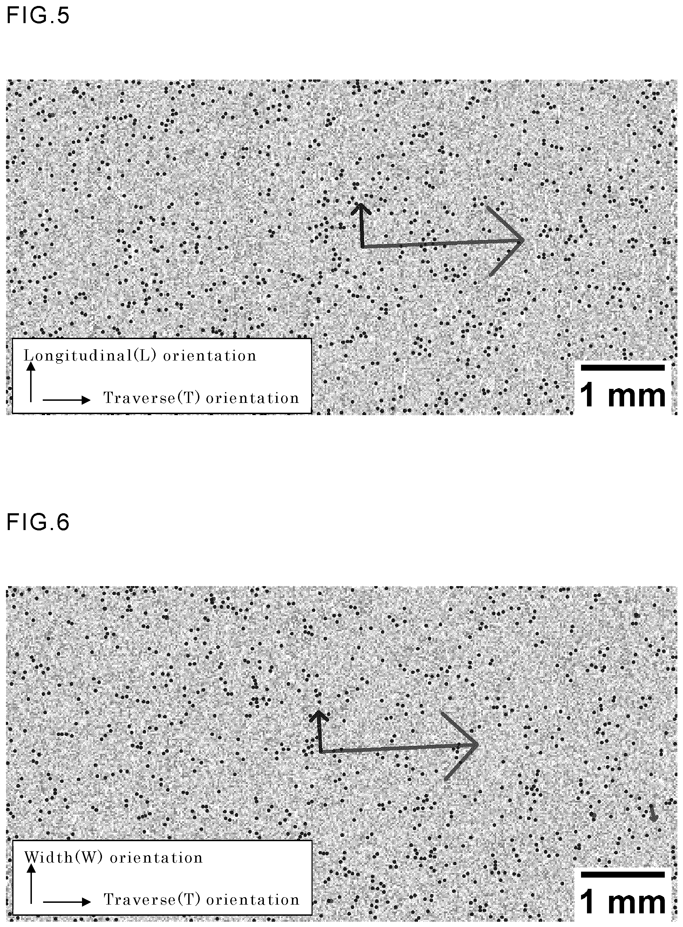

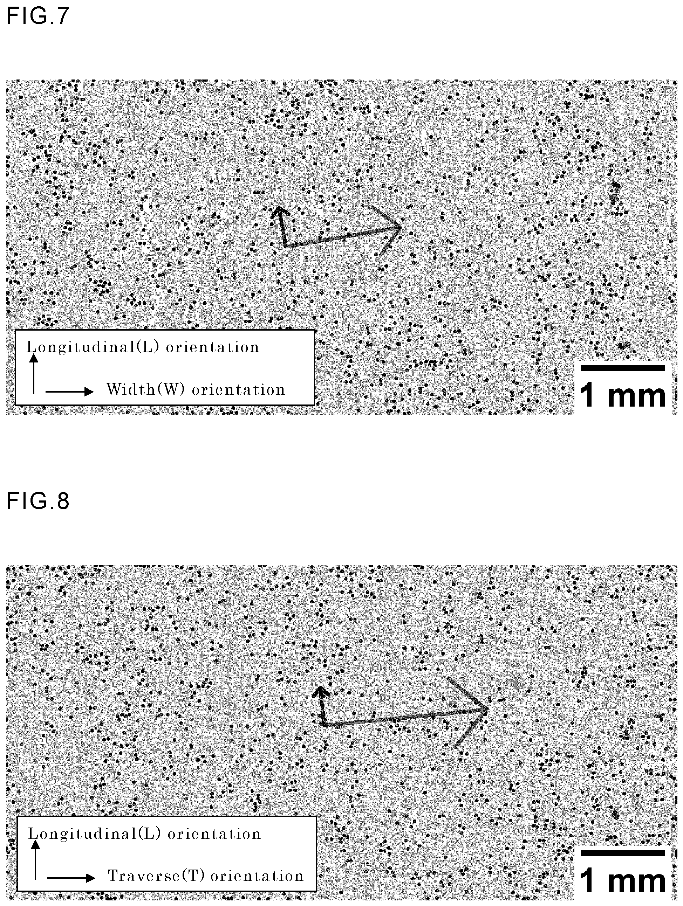

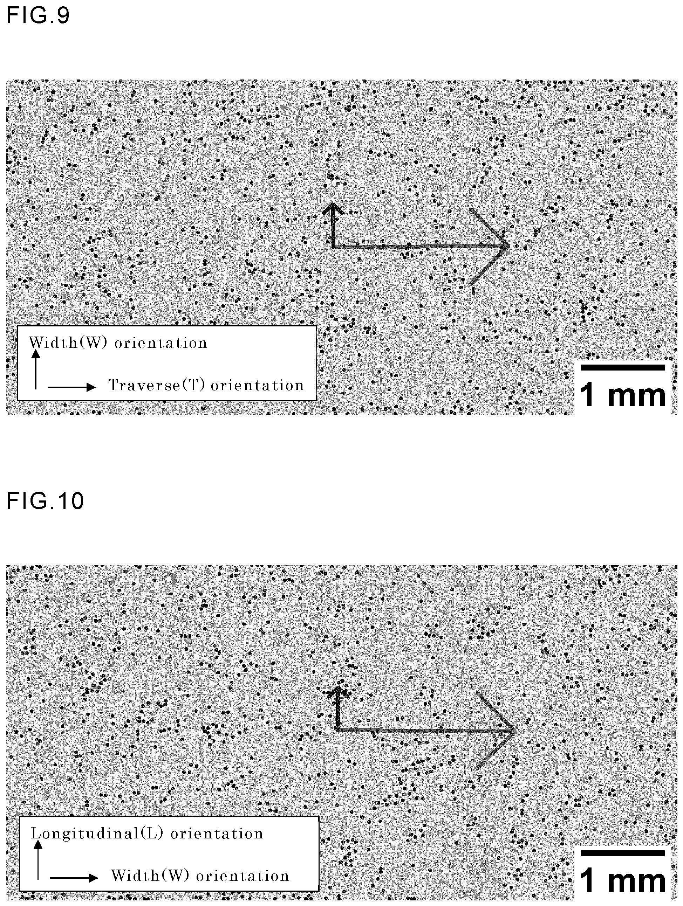

Next, binarization processing by a discriminant analysis method (Otsu method) was performed on the Ms-temperature mapping data after normalization processing. Then, main component analysis was performed on the Ms-temperature mapping data after binarization processing by using Python-OpenCV library. Images showing a first main component and a second main component obtained by the main component analysis are shown in FIGS. 5 to 10. A large arrow indicates the first main component and a small arrow indicates the second main component. Any of too small arrows was displayed as a point. Table 2 shows main components and respective contribution rates thereof in three directions of T-L, T-W, and W-L of Sample Nos. 1 and 2. When comparing the largest main component (eigenvalue) of each measurement surface, and second and third largest main components, the largest main component shows a value that is larger by one digit or more, and thus an attempt was made to compare Samples 1 and 2 by using the largest main component. The contribution rate of the first main component that is the largest main component was approximately 0.8 in the T-L direction and the T-W direction in Sample 1, but was as slightly low as 0.75 in the W-L direction. If the value is large, this case represents that the first main component is larger than the second main component in each measurement surface, and a bias is large.

Note that, in Sample 2, values of the first main component in all directions showed values larger as compared with Sample 1. It was confirmed that the contribution rate was approximately 0.8 in the T-L direction and the T-W direction, and the contribution rate in the W-L direction was 0.8 higher than 0.75 in Sample No. 1.

**[Table 2]**

| Sample | Direction | Maximum value of main component (eigenvalue) From first to third | First main component (a) | Second main component (b) | Contribution rate of first main component (a/(a + b)) | Contribution rate of second main component (b/(a + b)) |
|---|---|---|---|---|---|---|
| 1 | T-L | First | 191385 | 50692 | 0.791 | 0.209 |
| | | Second | 7847 | 856 | 0.902 | 0.098 |
| | | Third | 4055 | 1509 | 0.729 | 0.271 |
| | T-W | First | 186147 | 47751 | 0.796 | 0.204 |
| | | Second | 17683 | 974 | 0.948 | 0.052 |
| | | Third | 4922 | 1915 | 0.720 | 0.280 |
| | W-L | First | 138850 | 47908 | 0.743 | 0.257 |
| | | Second | 20098 | 6173 | 0.765 | 0.235 |
| | | Third | 9785 | 5299 | 0.649 | 0.351 |
| 2 | T-L | First | 193860 | 45111 | 0.811 | 0.189 |
| | | Second | 6051 | 4150 | 0.593 | 0.407 |
| | | Third | 3300 | 1743 | 0.654 | 0.346 |
| | T-W | First | 208265 | 51406 | 0.802 | 0.198 |
| | | Second | 1308 | 585 | 0.691 | 0.309 |
| | | Third | 1293 | 83 | 0.940 | 0.060 |
| | W-L | First | 210582 | 51290 | 0.804 | 0.196 |
| | | Second | 1578 | 262 | 0.857 | 0.143 |
| | | Third | 1464 | 205 | 0.877 | 0.123 |

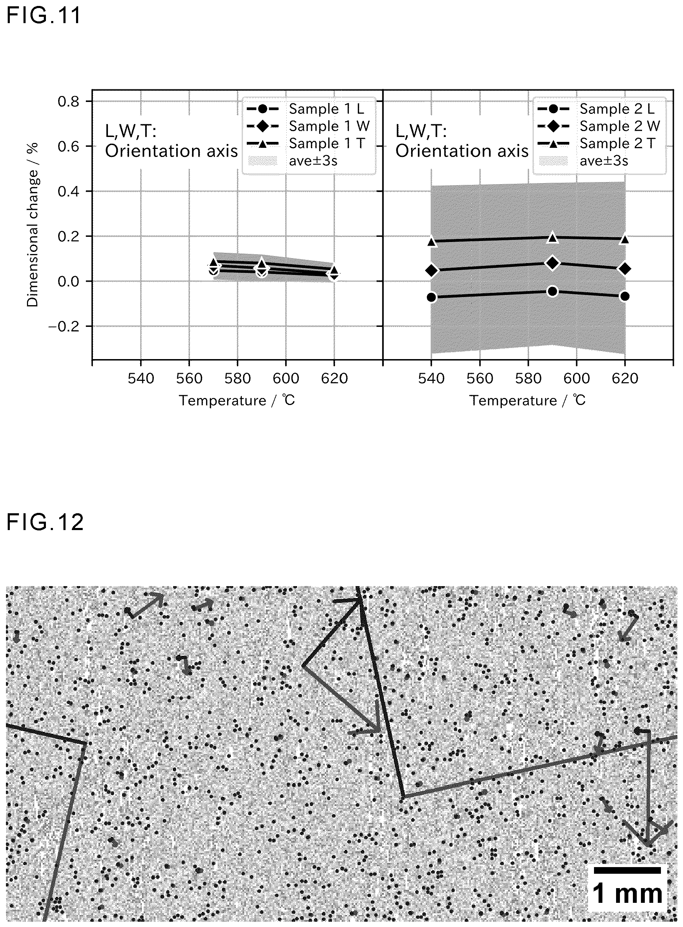

FIG. 11 is a graph of actually measured heat-treatment dimensional change. A left graph shows data of Sample No. 1 (Sample 1), and a right graph shows data of Sample No. 2 (Sample 2). The horizontal axis represents a tempering temperature. The vertical axis represents a dimensional change rate that is a ratio of dimensional change rate before and after a heat treatment. A positive value of the dimensional change rate indicates that dimensions increase due to the heat treatment, that is, expansion, and a negative value indicates that dimensions decrease due to the heat treatment, that is, shrinkage. A black circle indicates an actually measured value of a dimensional change rate in an L direction. A black rectangle indicates a dimensional change rate in a W direction. A black triangle indicates a dimensional change rate in a T direction. s in FIG. 11 is a standard deviation calculated from numerical values of the dimensional change rates at three points in the L, W, and T directions at each tempering temperature.

When comparing values of main components shown in Table 2 and actually measured values shown in FIG. 11 with each other, it could be confirmed that the magnitude of the first main component is related to the actual amount of dimensional change. From the above results, it was confirmed that according to the evaluation method of the disclosure, the bias or deviation of the Ms-temperature distribution resulting from segregation caused by a base composition can be evaluated as anisotropy of the dimensional change by applying the main composition analysis.

### Example 2

Steel ingots were prepared, and the steel ingots were subjected to hot forging, cooling, and annealing to obtain steel materials (annealed materials) of Sample Nos. 3 to 6 having compositions (analyzed values) shown in Table 3. The annealed materials were subjected to quenching treatment by semi-cooling for 10 minutes from 1020°C. Then, two times of tempering treatments were performed at 500°C after the quenching treatment. The numerical number in the half-cooling represents time required to reach a temperature (510°C) that is approximately the half of 1020°C.

**[Table 3]**

| (mass%) | | | | | | | | | | | | |
|---|---|---|---|---|---|---|---|---|---|---|---|---|
| Sample No. | Sample Cross-Sectional Dimension [mm] | C | Si | Mn | P | S | Ni | Cr | Mo | V | Al | Remainder |
| 3 | 205×475 | 0.66 | 1.48 | 0.75 | 0.022 | 0.054 | 0.09 | 4.91 | 1.48 | 0.15 | 0.067 | Fe and inevitable impurities |
| 4 | 205×475 | 0.60 | 1.47 | 0.75 | 0.021 | 0.050 | 0.08 | 4.86 | 1.43 | 0.15 | 0.069 | Fe and inevitable impurities |
| 5 | 37×425 | 0.65 | 1.37 | 0.81 | 0.025 | 0.053 | 0.10 | 4.89 | 1.43 | 0.14 | 0.061 | Fe and inevitable impurities |
| 6 | 27×375 | 0.64 | 1.37 | 0.80 | 0.025 | 0.053 | 0.10 | 4.91 | 1.40 | 0.14 | 0.060 | Fe and inevitable impurities |

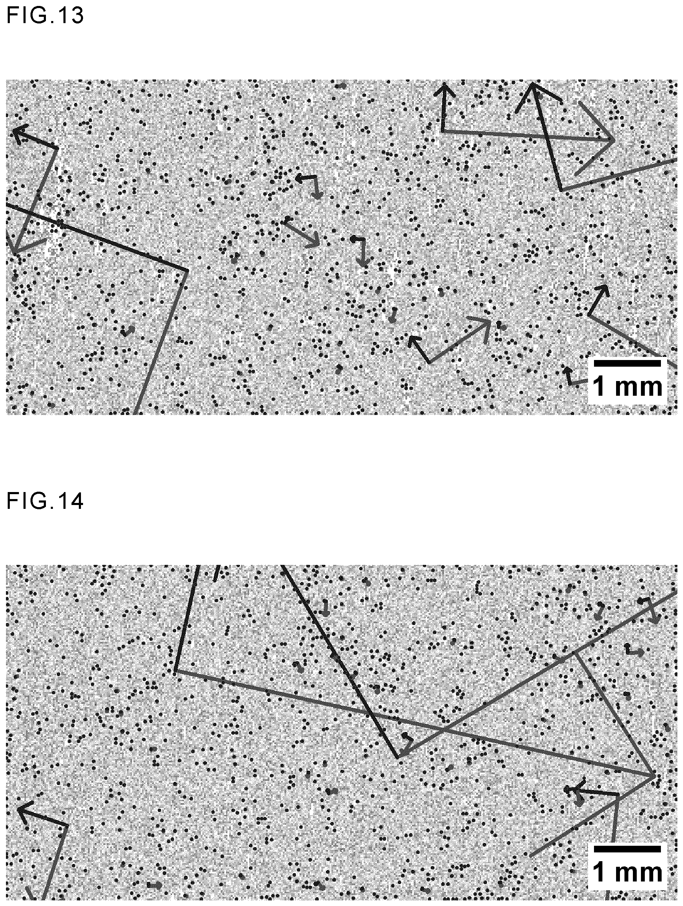

Samples having a length of 30 mm, a width of 25 mm, and a thickness of 20 mm were taken from each of the obtained quenched and tempered materials, and composition mapping data in a portion in the T-L direction shown in FIG. 4 was acquired by using FE-EPMA. Next, binarization processing by a discriminant analysis method (Otsu method) was performed on Ms-temperature mapping data after normalization processing. Then, main component analysis was performed on the Ms-temperature mapping data after binarization processing by using Python-OpenCV library. Images showing a first main component and a second main component obtained by the main component analysis are shown in FIGS. 12 to 15. A large arrow indicates the first main component and a small arrow indicates the second main component. Any of too small arrows was displayed as a point. Note that, FIGS. 12 to 15 are views in which the first main component is indicated by a red arrow and the second main component is indicated by a blue arrow in a similar manner as in FIG. 3. FIG. 4 shows main components and respective contribution rates thereof in the T-L direction of Sample Nos. 3 to 6. An attempt was made to evaluate the largest main component (eigenvalue) of each measurement surface, and then size dependency of steel material cross-sectional dimensions in the largest main component.

**[Table 4]**

| Sample | Direction | Maximum value of main component (eigenvalue) From first to third | First main component (a) | Second main component (b) | Contribution rate of first main component (a/(a + b)) | Contribution rate of second main component (b/(a + b)) |
|---|---|---|---|---|---|---|
| 3 | T-L | First | 114419 | 36385 | 0.759 | 0.241 |
| | | Second | 42096 | 16271 | 0.721 | 0.279 |
| | | Third | 13636 | 1609 | 0.894 | 0.106 |
| 4 | T-L | First | 54657 | 38925 | 0.584 | 0.416 |
| | | Second | 42388 | 13159 | 0.763 | 0.237 |
| | | Third | 20434 | 4008 | 0.836 | 0.164 |
| 5 | T-L | First | 98590 | 37140 | 0.726 | 0.274 |
| | | Second | 58472 | 17918 | 0.765 | 0.235 |
| | | Third | 22368 | 5197 | 0.811 | 0.189 |
| 6 | T-L | First | 213491 | 39285 | 0.845 | 0.155 |
| | | Second | 12301 | 6842 | 0.643 | 0.357 |
| | | Third | 3201 | 872 | 0.786 | 0.214 |

A contribution rate of the first main component that is the largest main component shows a value larger than that of the second main component in any sample. If the value is large, this case represents that the bias of the Ms-temperature distribution is large. From results in Table 4, it could be confirmed that the value of the first main component sequentially increases in the order of Sample 4, Sample 5, Sample 3, and Sample 6.

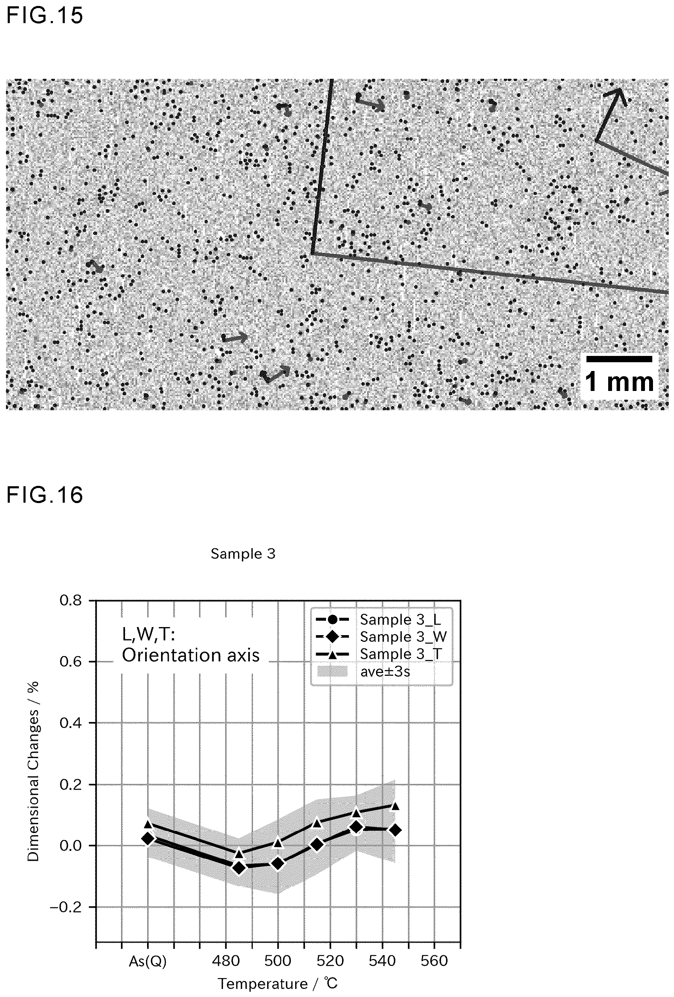

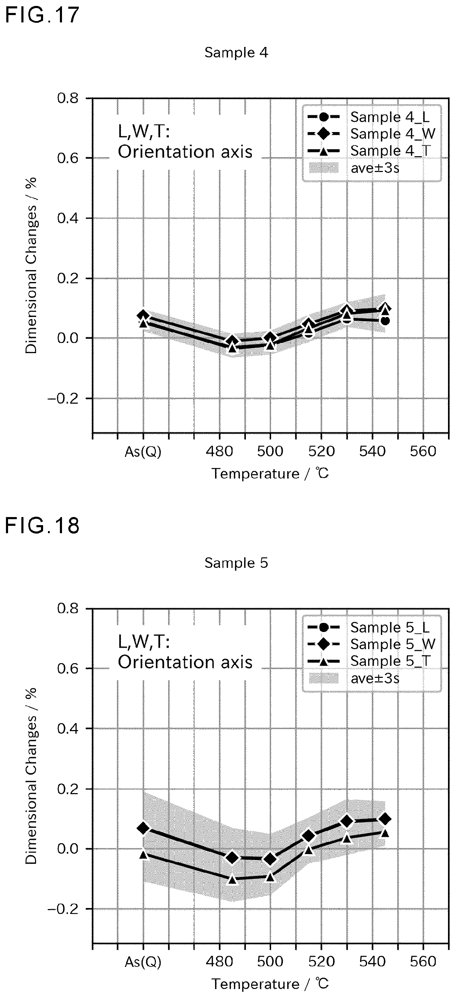

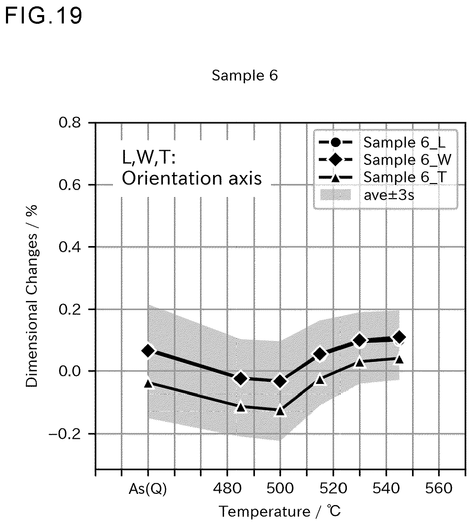

FIG. 16 is a graph of an actually measured value of the heat-treatment dimensional change of Sample No. 3 (Sample 3). FIG. 17 is a graph of an actually measured value of the heat-treatment dimensional change of Sample No. 4 (Sample 4). FIG. 18 is a graph of an actually measured value of the heat-treatment dimensional change of Sample No. 5 (Sample 5). FIG. 19 is a graph of an actually measured value of the heat-treatment dimensional change of Sample No. 6 (Sample 6). Respective samples are steel materials processed into a rectangular parallelepiped. In the graphs in FIGS. 16 to 19, the horizontal axis represents a tempering temperature, and the vertical axis represents a dimensional change rate. "As(Q)" in the horizontal axis represents that quenching was performed by semi-cooling for 10 minutes from 1020°C.

The dimensional change rate is indicated as a positive value in a case where dimensions representing expansion after the heat treatment increase and as a negative value in a case where dimensions representing shrinkage decrease. A black circle indicates an actually measured value of a dimensional change rate in the L direction. A black rectangle indicates a dimensional change rate in the W direction. A black triangle indicates a dimensional change rate in the T direction. s is a standard deviation calculated from numerical values of the dimensional change rates at three points in the L, W, and T directions at each tempering temperature.

When comparing values of the main components shown in Table 4 and actually measured values shown in FIGS. 16 to 19 with each other, it could be confirmed that biases of heat-treatment dimensional changes in the L, W, and T direction increase as a value of the first main component increases, and this was clear under a condition that the tempering temperature is 500°C or higher. It was also confirmed that a difference in dimensional change between Sample Nos. 3 and 5 is small because the contribution rates of the first main component are close to 0.759 and 0.726, respectively.

Here, Sample Nos. 3 and 5 are samples taken from a top side of the steel ingots, and Sample Nos. 4 and 6 are samples taken from a bottom side of the steel ingots. In this example, it is possible to stably obtain steel in which the dimensional change is suppressed by selecting the top side and the bottom side of the steel ingot on the basis of data capable of being obtained through the main component analysis. In addition, even in a material in which the contribution rate of the first main component is large (a difference between the first main component and the second main component is large), it is possible to expect improvement in the dimensional change characteristics when performing component adjustment so that or adjustment of manufacturing conditions after ingot making so as to reduce the contribution rate of the first main component.

### Example 3

A coil having a wire diameter of 5 mm was prepared from an ingot of a high-speed tool steel (high-speed steel) by hot working. Description will be given with reference to coils of Sample Nos. 7, 12, and 14. Heat treatment conditions when preparing the coils include holding at 1190°C for 15 minutes, and rapid cooling under a nitrogen gas pressure of 5 bar. Analyzed values of a composition of the steel ingot are shown in Table 5. The analyzed values are values obtained by extracting and analyzing a part of a molten metal immediately before tapping after ladle refining.

**[Table 5]**

| | | | | | | | | (mass%) |
|---|---|---|---|---|---|---|---|---|
| C | Si | Mn | Cr | W | Mo | V | Co | Remainder |
| 1.07 | 0.26 | 0.28 | 3.87 | 1.42 | 9.27 | 1.12 | 7.76 | Fe and inevitable impurities |

Samples were respectively taken from a portion corresponding to a top side of the steel ingot and a portion corresponding to a bottom side of the steel ingot in the prepared coil. The samples were cut along a longitudinal direction of the wire so as to include a wire diameter center axis, and a cut surface was analyzed by the FE-EPMA. In the following description, B at the end of Sample No. represents data of a sample taken from the bottom side, and T at the end of Sample No. represents data of a sample taken from the top side of the steel ingot.

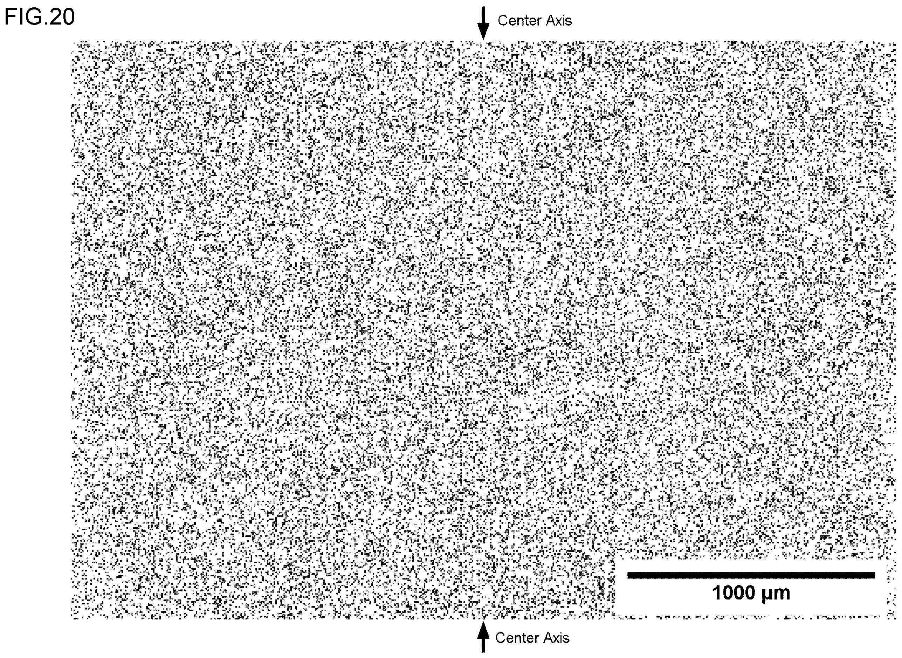

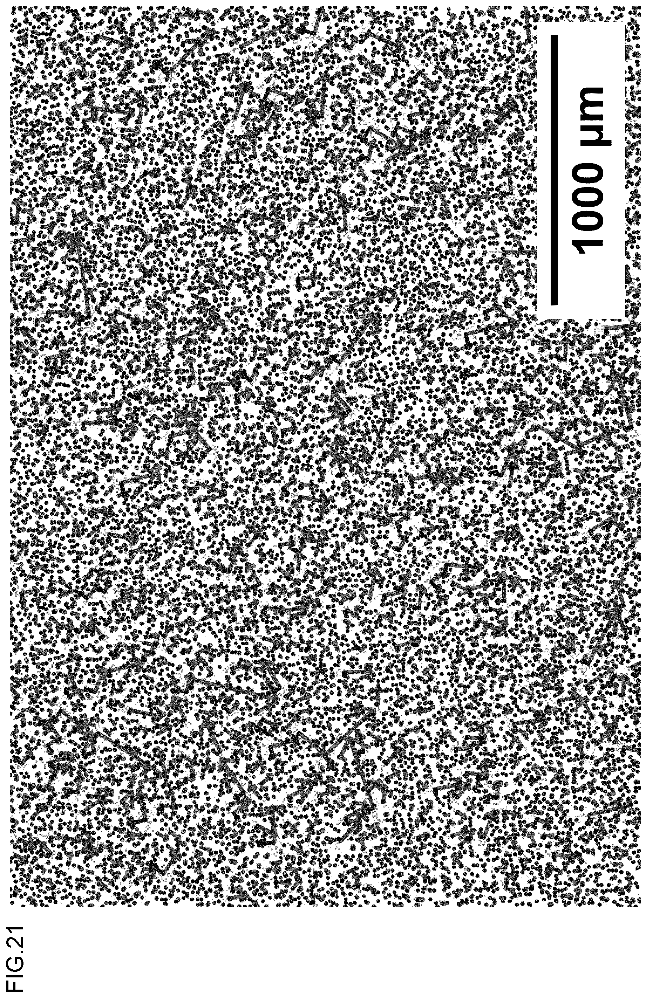

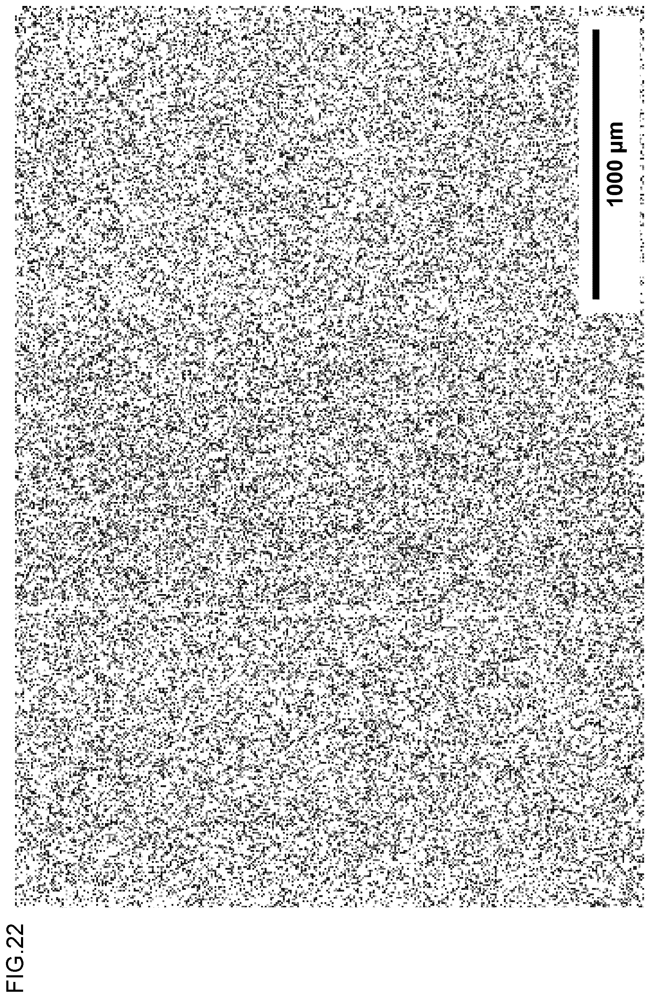

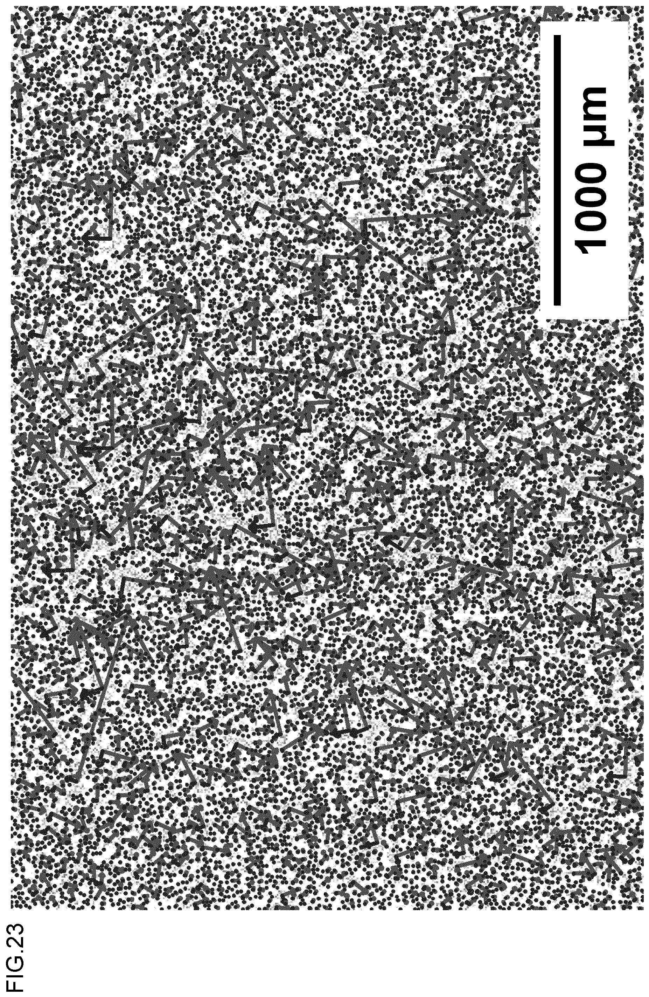

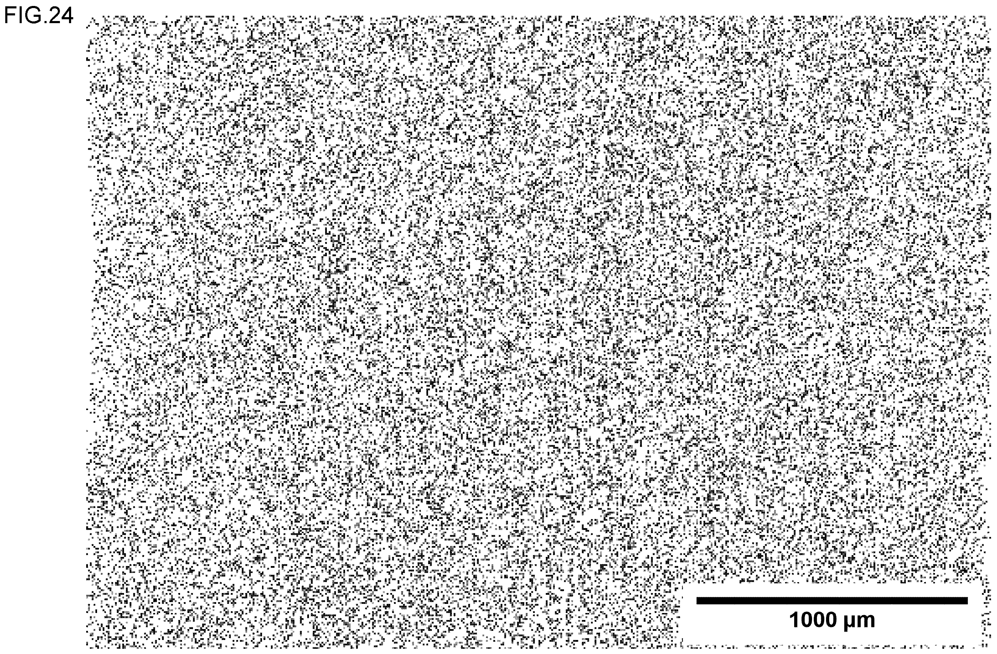

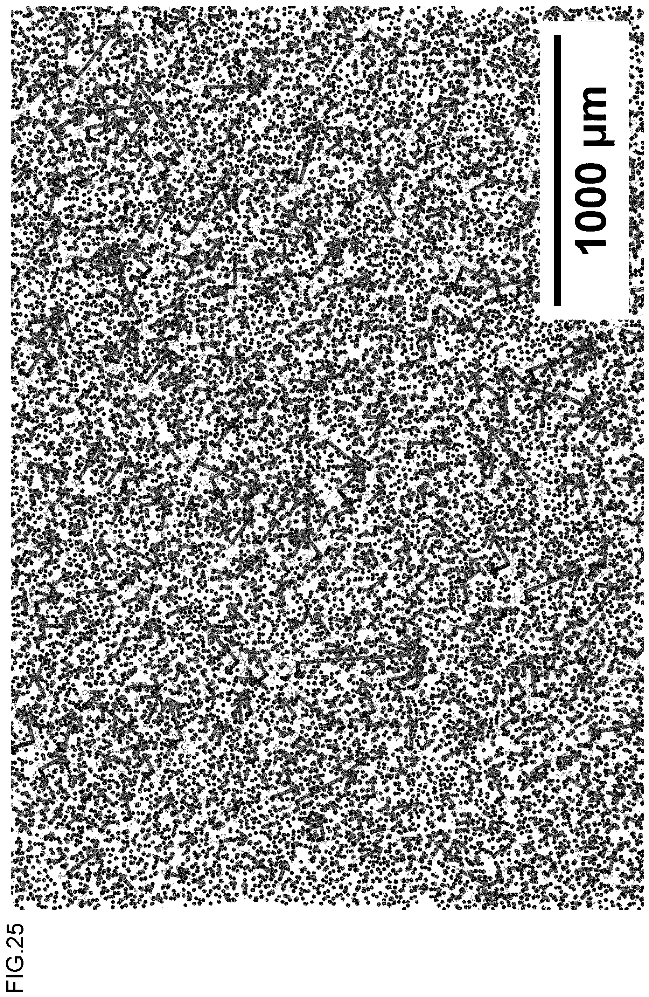

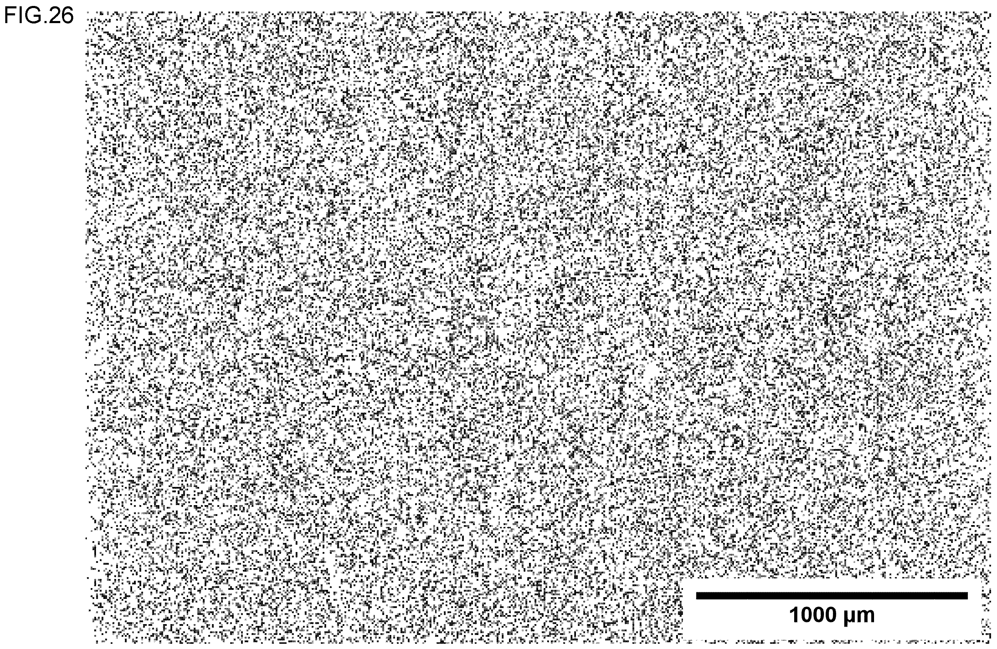

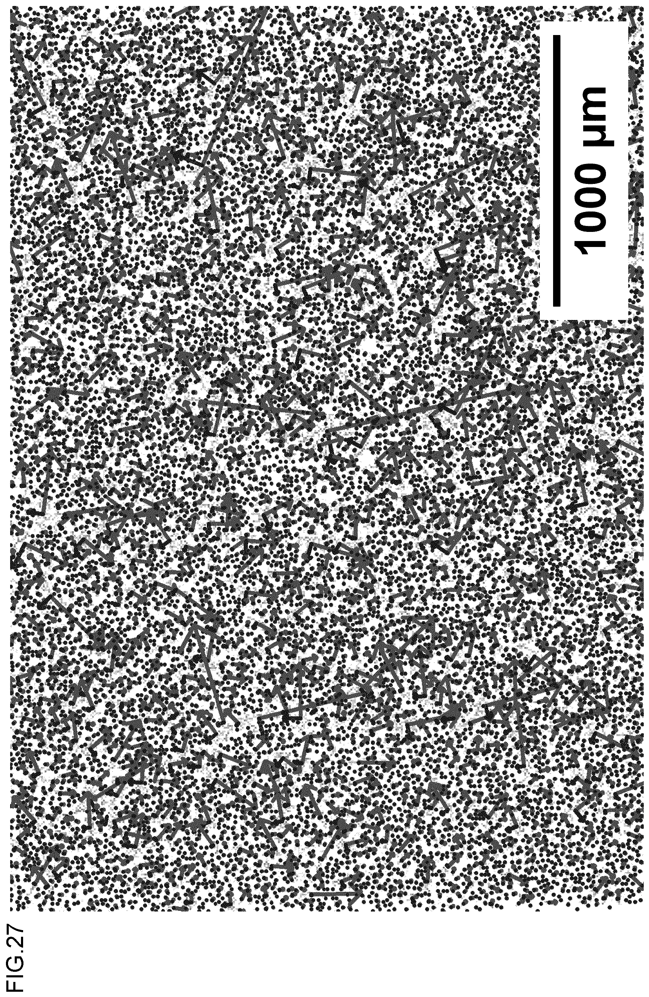

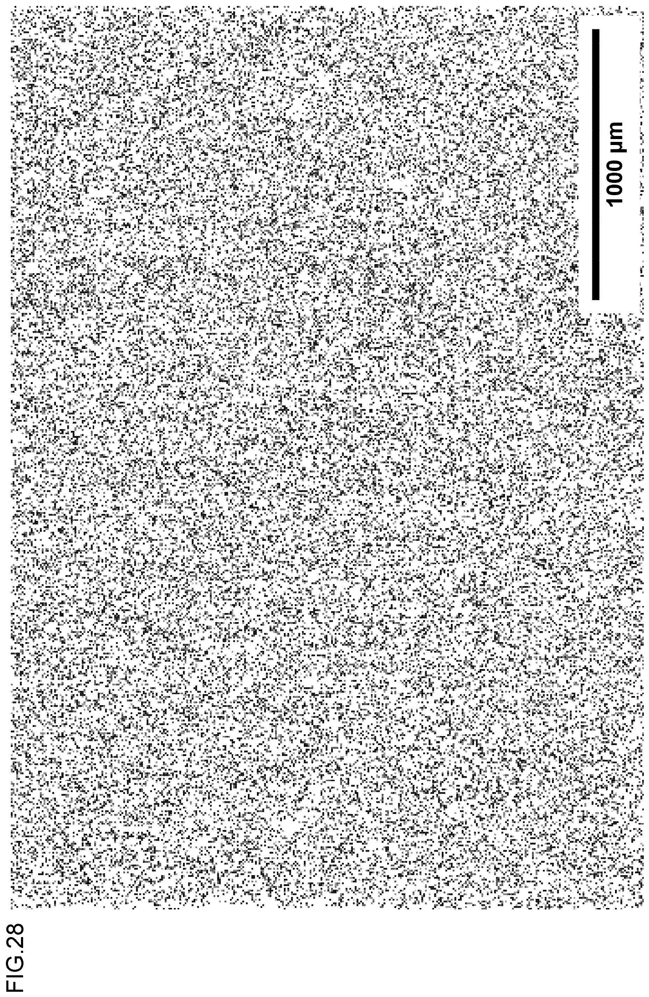

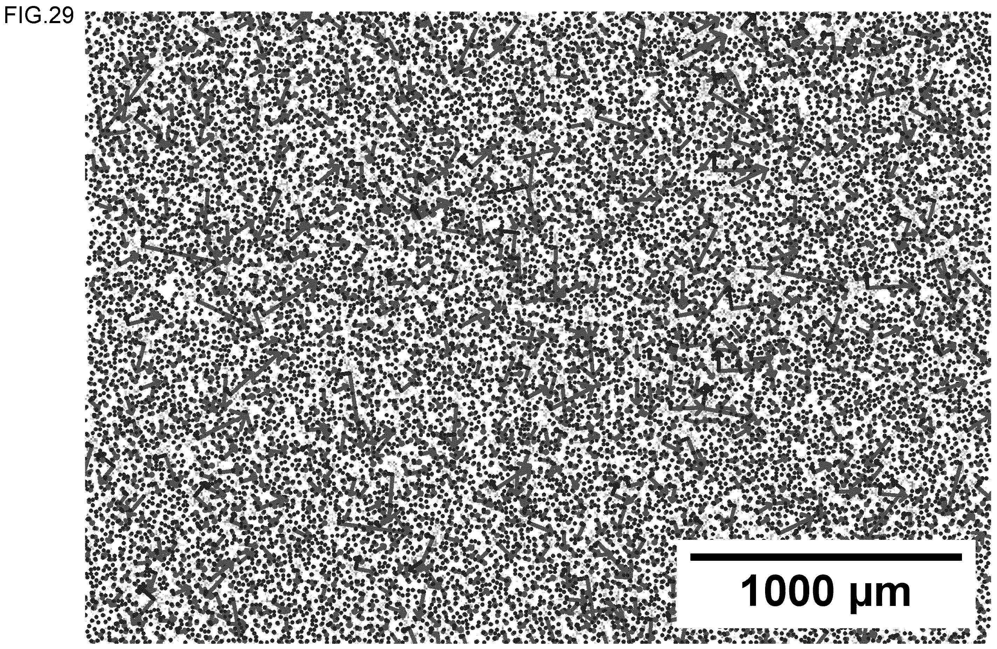

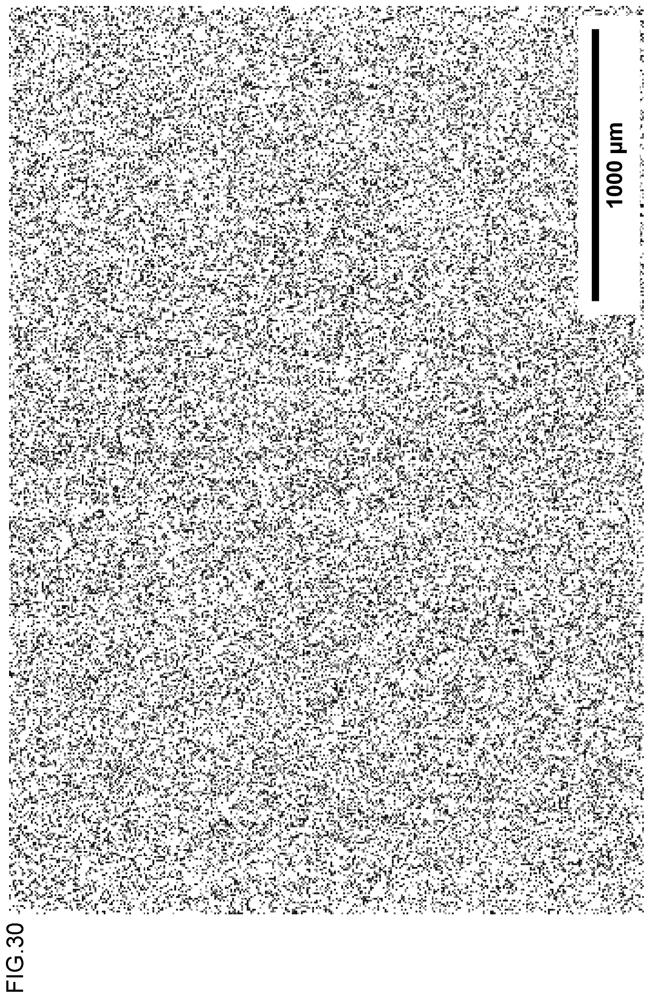

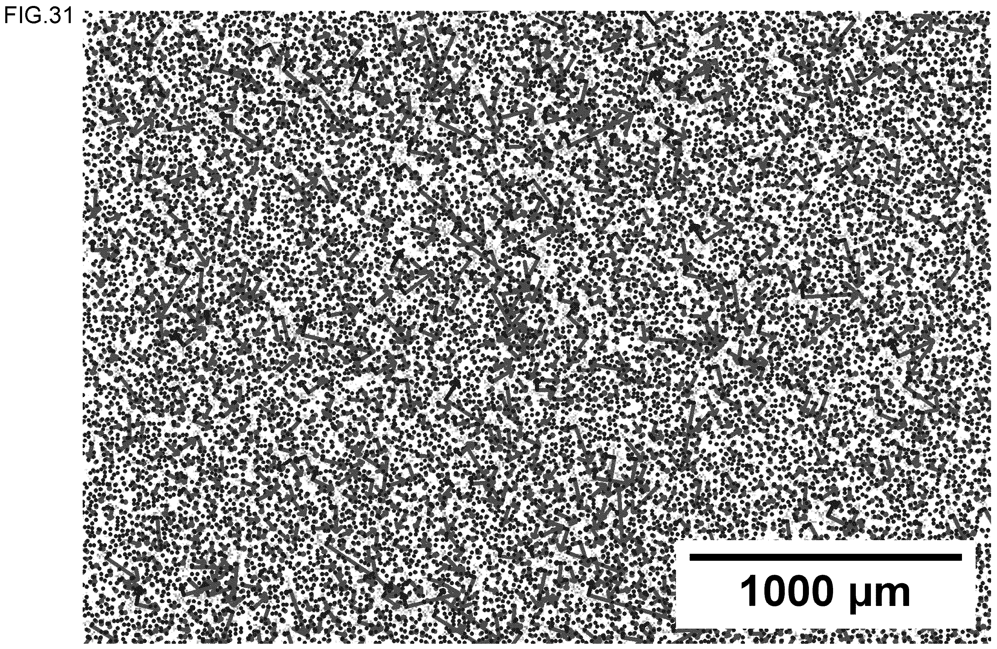

FIG. 20 is an Ms-temperature mapping image of Sample No. 7B. A wire diameter center axis is indicated by an arrow. FIG. 21 is a view illustrating main component analysis results of Sample No. 7B. FIG. 22 is an Ms-temperature mapping image of Sample No. 7T. FIG. 23 is a view illustrating main component analysis results of Sample No. 7T. FIG. 24 is an Ms-temperature mapping image of Sample No. 12B. FIG. 25 is a view illustrating main component analysis results of Sample No. 12B. FIG. 26 is an Ms-temperature mapping image of Sample No. 12T. FIG. 27 is a view illustrating main component analysis results of Sample No. 12T. FIG. 28 is an Ms-temperature mapping image of Sample No. 14B. FIG. 29 is a view illustrating main component analysis results of Sample No. 14B. FIG. 30 is an Ms-temperature mapping image of Sample No. 14T. FIG. 31 is a view illustrating main component analysis results of Sample No. 14T.

With regard to the Ms-temperature mapping images shown in FIG. 20 and the like, an outline of a creating method will be described. After mirror polishing the cut surface, a composition of main elements was acquired as mapping data by using the FE-EPMA. The number of imaging pixels of a compositional image is 500 pixels × 350 pixels. Then, in a state in which the number of pixels of data was enlarged by four times vertically and horizontally, mapping data of Ms-temperature was obtained by using a calculation method of an Ms-temperature prediction formula. The state of being enlarged four times vertically and horizontally represents that 1 pixel is enlarged isotropically in the vertical and horizontal direction to 4 pixels (vertical) and 4 pixels (horizontal). The Ms-temperature mapping images shown in FIG. 20 and the like are 24-bit color images of 2000 pixels × 1400 pixels. A scale of 1000 µm is shown on a lower-right side in the respective drawings.

The main component analysis results shown in FIG. 21 and the like show results obtained by calculating the first main component and the second main component in the Ms-temperature after conducting normalization processing and binarization processing on the Ms-temperature mapping images. Black and white image are shown due to limitation of electronic application software used for international patent application, but actually, as in FIG. 3, the drawings are views in which the first main component at each measurement point is indicated by a red arrow and the second main component at each measurement point is indicated by a blue arrow. Due to a difference in sample characteristics, measurement conditions, and the like, the main component analysis results of this example as shown in FIG. 21 and the like are indicated by a larger number of arrows as compared with the main component analysis results of Examples 1 and 2 as shown FIG. 3 and the like.

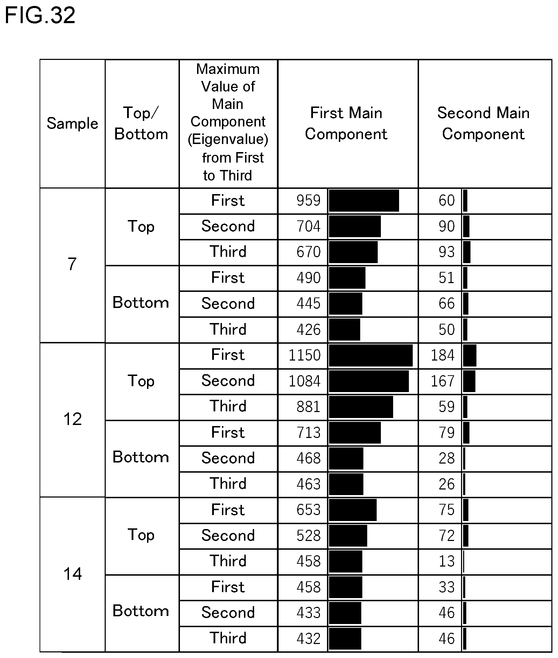

FIG. 32 is an explanatory view illustrating evaluation results of main components. An outline of data will be described. With respect to respective measurement points of the main component analysis results, a magnitude of an eigenvector obtained by combining a vector corresponding to the first main component and a vector corresponding to the second main component was calculated. Then, with respect to respective samples, data of three measurement points with a large eigenvector was extracted. A value of the first main component and a value of the second main component for the extracted three measurement points are shown in FIG. 32 in a table form. Further, on the right side of a value of the first main component and a value of the second main component, a bar having a length proportional to each value is displayed for intuitive understanding.

From FIG. 32, when comparing the top side and the bottom side, it can be seen that the first main component tends to be larger on the top side. This is consistent with a technical common sense that a cooling rate on the top side is slow during casting, and thus segregation is likely to occur and anisotropy in material characteristics becomes stronger.

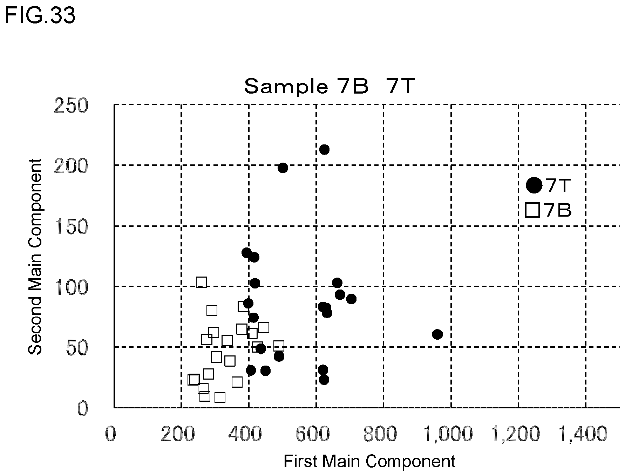

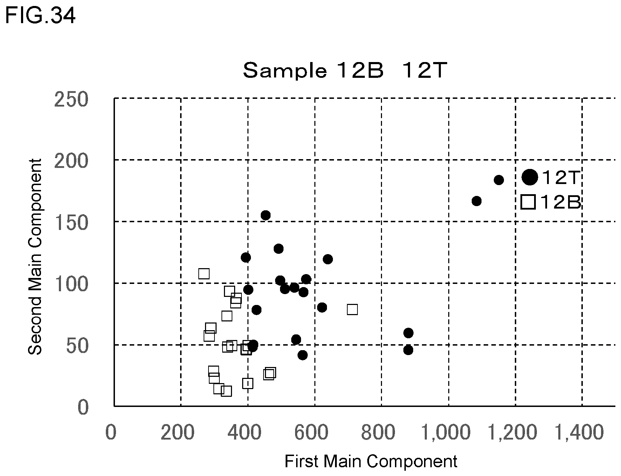

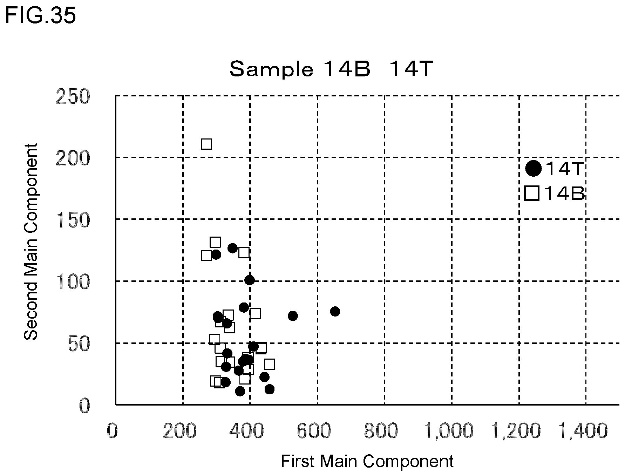

FIG. 33 is a scatter plot of a main component of Sample No. 7 (Sample 7). FIG. 34 is a scatter plot of a main component of Sample No. 12 (Sample 12). FIG. 35 is a scatter plot of a main component of Sample No. 14 (Sample 14). In FIGS. 33 to 35, the horizontal axis represents a value of the first main component and the vertical axis represents a value of the second main component. With respect to respective samples, 20 measurement points extracted from points with a larger eigenvector for each of the top side and the bottom side are plotted. A square indicates data on the bottom (B) side, and a black circle indicates data of the top (T) side.

From FIGS. 33 to 35, it can be seen that a difference between the top side and the bottom side is large in Sample Nos. 7 and 12, and a difference between the top side and the bottom side is small in Sample No. 14.

As described above, it is possible to discriminate a material taken from a top side portion of a steel ingot and a material taken from a bottom side portion of the steel ingot on the basis of the magnitude of the first main component and the second main component at measurement points with large eigenvectors. Specifically, it is possible to determine that a position where a material with large first and second main components is taken is the top portion of the steel ingot. Furthermore, it is also possible to identify a small-sized steel ingot in which a cooling rate during solidification is fast and a large-sized steel ingot in which the cooling rate is low from each other on the basis of a distribution state of the first main component and the second main component.

For example, for applications requiring a steel material with weak anisotropy, a steel ingot with a first main component smaller than a predetermined specified value may be used, and for applications requiring a steel material with strong anisotropy without a problem, a steel ingot with the first main component equal to or larger than a predetermined specified value may be used. By selecting a steel ingot shape corresponding to an application, a reduction in the manufacturing cost and securement of necessary quality are compatible with each other.

For example, by adjusting manufacturing conditions so that the first main component on the top side is smaller than the first main component on the bottom side, a steel ingot with uniform characteristics can be manufactured.

Note that, it could be confirmed that the Ms-temperature mapping and the main component analysis can be performed in a similar procedure even when changing the irradiation current during the FE-EPMA is changed from 0.1 µA to 0.5 µA. When enlarging the irradiation current, an improvement in data accuracy can be expected. In addition, the data accuracy can also be improved by increasing the density of the measurement points during FE-EPMA, that is, by increasing resolution.

### [Embodiment 2]

This embodiment relates to a program that is used for steel evaluation. Description of a portion common to Embodiment 1 will be omitted.

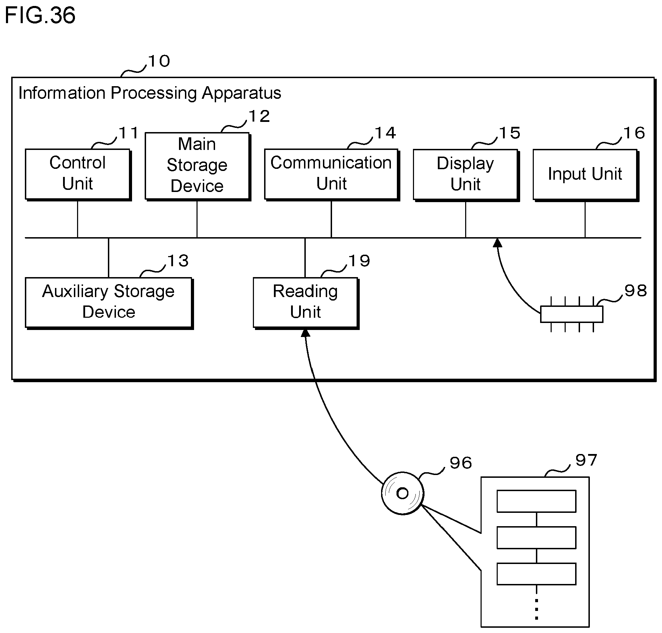

FIG. 36 is an explanatory view illustrating a configuration of an information processing apparatus 10. The information processing apparatus 10 includes a control unit 11, a main storage device 12, an auxiliary storage device 13, a communication unit 14, a display unit 15, an input unit 16, and a bus.

The control unit 11 is an operation control device that executes a program of this embodiment. One or more central processing units (CPUs), graphics processing units (GPUs), multi-core CPUs, or the like are used in the control unit 11. The control unit 11 is connected to respective hardware portions constituting the information processing apparatus 10 through the bus.

The main storage device 12 is a storage device such as static random access memory (SRAM), a dynamic random access memory (DRAM), and a flash memory. The main storage device 12 temporarily stores information necessary during processing performed by the control unit 11 and a program that is being executed by the control unit 11.

The auxiliary storage device 13 is a storage device such as an SRAM, a flash memory, a hard disk, and a magnetic tape. The auxiliary storage device 13 stores a program to be executed by the control unit 11, and various pieces of data necessary for execution of the program.

The communication unit 14 is an interface that performs communication between the information processing apparatus 10 and a network. The display unit 15 is, for example, a liquid crystal display panel or an organic electro-luminescence (EL) panel. The input unit 16 is, for example, an input device such as a keyboard, a mouse, and a microphone. The display unit 15 and the input unit 16 may be stacked to constitute a touch panel.

A portable recording medium 96 is, for example, a universal serial bus (USB) memory, a compact disc read only memory (CD-ROM), a magneto-optical disc medium, another optical disc medium, an SD memory card, or the like. A program 97 to be described later is stored in the portable recording medium 96.

A reading unit 19 is, for example, an interface such as a USB connector, a CD-ROM driver, an SD memory reader capable of being connected to the portable recording medium 96. A semiconductor memory 98 is a memory that stores the program 97, and can be attached to the inside of the information processing apparatus 10.

The information processing apparatus 10 is a general-purpose personal computer, a tablet, a large-scaled computing machine, a virtual machine operating on the large-scaled computing machine, or a quantum computer. The information processing apparatus 10 may be configured by hardware such as a plurality of personal computers or a large-scaled computing machine that performs distributed processing. The information processing apparatus 10 may be configured by a cloud computing system. The information processing apparatus 10 may be configured by hardware such as a plurality of personal computers or a large-scaled computing machine that operates in cooperation.

The program 97 is recorded on the portable recording medium 96. The control unit 11 reads out the program 97 through the reading unit 19, and stores the program 97 in the auxiliary storage device 13. In addition, the control unit 11 may read out the program 97 stored in the semiconductor memory 98. In addition, the control unit 11 may download the program 97 from a server computer (not illustrated) connected to the control unit 11 through the communication unit 14 and a network (not illustrated) and may store the program 97 in the auxiliary storage device 13.

The program 97 is installed as a control program of the information processing apparatus 10, and is loaded in the main storage device 12 to be executed. The program 97 in this embodiment is an example of a program product.

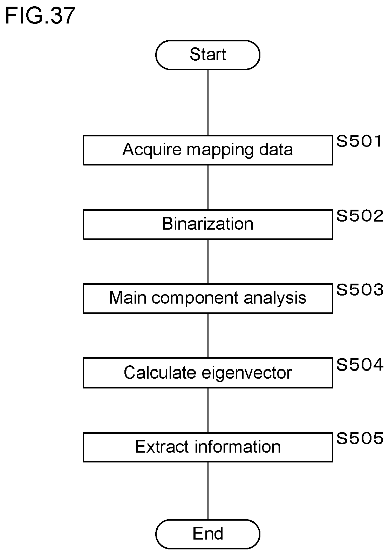

FIG. 37 is a flowchart illustrating a processing flow of the program 97. The control unit 11 acquires mapping data of a base composition (step S501). The mapping data is created by an electron probe micro analyzer (not illustrated), and is stored in the auxiliary storage device 13 or an external large-capacity device (not illustrated). The electron probe micro analyzer and the information processing apparatus 10 are connected to each other through the network, and the control unit 11 may acquire mapping data directly from the electron probe micro analyzer.

The control unit 11 performs binarization processing of the mapping data (step S502). As described above, the known Otsu method can be used in the binarization processing. The control unit 11 performs main component analysis (step S503). As described above, the main component analysis can be executed, for example, by using Python-OpenCV library.

The control unit 11 calculates an eigenvector that is a composed vector of a first main component vector and a second main component vector for each set of the first main component and the second main component (step S504). The control unit 11 extracts information on a predetermined number of eigenvectors in a descending order (step S505). Examples of the information on the eigenvector include information in a position where the eigenvector is calculated in the mapping data, the direction and magnitude of the first main component vector, and the direction and magnitude of the second main component vector. The control unit 11 terminates the processing.

For example, the control unit 11 may acquire information on three eigenvectors in a descending order in step S505, and may display a table shown in FIG. 32 on the display unit 15. FIG. 32 can be created by results obtained by executing the program in FIG. 37 six times by using a total of six pieces of mapping data for the top and bottom of each of three samples.

For example, the control unit 11 may acquire information on 20 eigenvectors in a descending order in step S505, and may display scatter plots shown in FIGS. 33 to 35 on the display unit 15. FIGS. 33 to 35 can be created by results obtained by executing the program in FIG. 37 twice by using a total of two pieces of mapping data for the top and bottom of one sample.

The control unit 11 may display whether or not the magnitude of the first main component vector or the contribution rate of the first main component vector is larger than a predetermined threshold value on the display unit 15 on the basis of eigenvectors extracted from step S505. The control unit 11 may transmit information to information device such as a smartphone through the network instead of displaying the information on the display unit 15.

The technical characteristics (configuration requirements) described in the respective examples can be combined with each other, and new technical characteristics can be formed through the combination.

The embodiments disclosed herein should be considered as illustrative in all respects and not restrictive. The scope of the invention is represented by the appended claims rather than being limited to the above description.

### Reference Signs List

- 10: information processing apparatus
- 11: control unit
- 12: main storage device
- 13: auxiliary storage device
- 14: communication unit
- 15: display unit
- 16: input unit
- 19: reading unit
- 96: portable recording medium
- 97: program
- 98: semiconductor memory

## Claims

1. A steel evaluation method comprising:
a process of measuring a surface of steel with an electron probe micro analyzer to obtain mapping data of a base composition;
a process of obtaining mapping data of an Ms temperature from the mapping data of the base composition (S501);
a process of binarizing the mapping data of the Ms temperature (S502);
a process of obtaining a first main component and a second main component by main component analysis of the mapping data of the Ms temperature which has been subjected to the binarization processing (S503); and
a process of evaluating dimensional change characteristics of the steel on the basis of a relationship between the first main component and the second main component.

2. The steel evaluation method according to claim 1, further comprising:
a process of calculating a difference between the first main component and the second main component with respect to each of a plurality of steel ingots; and
a process of evaluating a steel ingot having a larger difference as a steel ingot having a larger variation in the dimensional change characteristics.

3. The steel evaluation method according to claim 1 or 2, further comprising:
a process of calculating a contribution rate of the first main component for the sum of the first main component and the second main component with respect to each of the plurality of steel ingots; and
a process of selecting a steel ingot in which the contribution rate of the first main component is lower from the plurality of steel ingots.

4. The steel evaluation method according to any of claims 1 to 3, further comprising:
with respect to the first main component and the second main component obtained with respect to samples taken from a plurality of sites of each of the steel ingots,
a process of calculating a magnitude of an eigenvector obtained by combining a vector corresponding to the first main component and a vector corresponding to the second main component (S504);
a process of sequentially extracting the eigenvector in a descending order; and
a process of extracting the first main component corresponding to the extracted eigenvector,
wherein a position, from which the sample in which the first main component is large is taken, is determined as a top side of the steel ingot.

5. A program causing a computer (10) to execute processing of:
acquiring mapping data of a base composition which has been created by measuring a surface of steel with an electron probe micro analyzer (S501);
creating mapping data of an Ms temperature from the mapping data of the base composition;
binarizing the mapping data of the Ms temperature (S502);
calculating a first main component and a second main component by main component analysis of the mapping data of the Ms temperature which has been subjected to the binarization processing (S503); and
evaluating dimensional change characteristics of the steel on the basis of a relationship between the first main component and the second main component.

## Patentansprüche

1. Ein Verfahren zur Bewertung von Stahl, das Folgendes aufweist:
einen Prozess des Messens einer Oberfläche von Stahl mit einem Elektronenstrahl-Mikroanalysator, um Mapping-Daten einer Basiszusammensetzung zu erhalten;
einen Prozess des Erhaltens von Mapping-Daten einer Ms-Temperatur aus den Mapping-Daten der Basiszusammensetzung (S501);
einen Prozess des Binärisierens der Mapping-Daten der Ms-Temperatur (S502);
einen Prozess des Erhaltens einer ersten Hauptkomponente und einer zweiten Hauptkomponente durch Hauptkomponentenanalyse der Mapping-Daten der Ms-Temperatur, die der Binärisierungsverarbeitung unterzogen worden sind (S503); und
einen Prozess des Bewertens von Dimensionsänderungs-Eigenschaften des Stahls auf der Grundlage einer Beziehung zwischen der ersten Hauptkomponente und der zweiten Hauptkomponente.

2. Das Verfahren zur Bewertung von Stahl nach Anspruch 1, das ferner Folgendes aufweist:
einen Prozess des Berechnens einer Differenz zwischen der ersten Hauptkomponente und der zweiten Hauptkomponente in Bezug auf jeden von einer Vielzahl von Stahlingots bzw. -blöcken; und
einen Prozess des Bewertens eines Stahlingots bzw. -blocks mit einer größeren Differenz als ein Stahlingot bzw. -block mit einer größeren Variation hinsichtlich der Dimensionsänderungs-Eigenschaften.

3. Das Verfahren zur Bewertung von Stahl nach Anspruch 1 oder 2, das ferner Folgendes aufweist:
einen Prozess des Berechnens eines Beitragsgrads der ersten Hauptkomponente für die Summe der ersten Hauptkomponente und der zweiten Hauptkomponente in Bezug auf jeden der Vielzahl von Stahlblöcken; und
einen Prozess des Auswählens eines Stahlblocks, bei dem der Beitragsgrad der ersten Hauptkomponente niedriger ist, aus der Vielzahl von Stahlblöcken.

4. Das Verfahren zur Bewertung von Stahl nach einem der Ansprüche 1 bis 3, das ferner Folgendes aufweist:
und zwar in Bezug auf die erste Hauptkomponente und die zweite Hauptkomponente, die in Bezug auf Proben erhalten wurden, die von einer Vielzahl von Stellen jedes der Stahlblöcke entnommen werden,
einen Prozess des Berechnens einer Größe eines Eigenvektors, der durch Kombinieren eines der ersten Hauptkomponente entsprechenden Vektors und eines der zweiten Hauptkomponente entsprechenden Vektors erhalten wurde (S504);
einen Prozess des sequentiellen Extrahierens des Eigenvektors in einer absteigenden Reihenfolge; und
einen Prozess des Extrahierens der ersten Hauptkomponente, die dem extrahierten Eigenvektor entspricht,
wobei eine Position, von der die Probe, bei der die erste Hauptkomponente groß ist, entnommen wurde, als eine Oberseite des Stahlblocks bestimmt wird.

5. Ein Programm, das einen Computer (10) dazu veranlasst, Folgendes auszuführen:
Erhalten von Mapping-Daten einer Basiszusammensetzung, die durch Messen einer Oberfläche von Stahl mit einem Elektronenstrahl-Mikroanalysator erstellt worden sind (S501);
Erstellen von Mapping-Daten einer Ms-Temperatur aus den Mapping-Daten der Basiszusammensetzung;
Binärisieren der Mapping-Daten der Ms-Temperatur (S502);
Berechnen einer ersten Hauptkomponente und einer zweiten Hauptkomponente durch Hauptkomponentenanalyse der Mapping-Daten der Ms-Temperatur, die dem Prozessschritt der Binärisierung unterzogen worden sind (S503); und
Bewerten von Dimensionsänderungs-Eigenschaften des Stahls auf der Grundlage einer Beziehung zwischen der ersten Hauptkomponente und der zweiten Hauptkomponente.

## Revendications

1. Procédé d'évaluation de l'acier comprenant :
un processus de mesure d'une surface d'acier à l'aide d'un microanalyseur à sonde électronique pour obtenir des données de cartographie d'une composition de base ;
un processus d'obtention de données cartographiques de la température Ms à partir des données cartographiques de la composition de base (S501) ;
un processus de binarisation des données cartographiques de la température Ms (S502) ;
un processus d'obtention d'un premier composant principal et d'un deuxième composant principal par analyse en composants principaux des données de cartographie de la température Ms qui ont été soumises au processus de binarisation (S503) ; et
un processus d'évaluation de caractéristiques de variation dimensionnelle de l'acier sur la base d'une relation entre la première composante principale et la deuxième composante principale.

2. Procédé d'évaluation de l'acier selon la revendication 1, comprenant en outre :
un processus de calcul d'une différence entre la première composante principale et la deuxième composante principale pour chacun d'une pluralité de lingots d'acier ; et
un processus d'évaluation d'un lingot d'acier présentant une différence plus importante comme étant un lingot d'acier présentant une variation plus importante des caractéristiques de variation dimensionnelle.

3. Procédé d'évaluation de l'acier selon la revendication 1 ou 2, comprenant en outre :
un processus de calcul d'un taux de contribution de la première composante principale à la somme de la première composante principale et de la deuxième composante principale pour chacun des lingots d'acier de la pluralité ; et
un processus de sélection, parmi la pluralité de lingots d'acier, d'un lingot d'acier dans lequel le taux de contribution de la première composante principale est plus faible.

4. Procédé d'évaluation de l'acier selon l'une quelconque des revendications 1 à 3, comprenant en outre :
s'agissant de la première composante principale et de la deuxième composante principale obtenues à partir d'échantillons prélevés sur une pluralité d'emplacements de chacun des lingots d'acier,
un processus de calcul de l'amplitude d'un vecteur propre obtenu en combinant un vecteur correspondant à la première composante principale et un vecteur correspondant à la deuxième composante principale (S504) ;
un processus d'extraction séquentielle du vecteur propre par ordre décroissant ; et
un processus d'extraction de la première composante principale correspondant au vecteur propre extrait,
dans lequel une position, à partir de laquelle est prélevé l'échantillon dans lequel la première composante principale est importante, est déterminée comme étant la face supérieure du lingot d'acier.

5. Programme amenant un ordinateur (10) à exécuter un traitement de :
acquisition de données de cartographie d'une composition de base qui ont été créées en mesurant une surface d'acier à l'aide d'un microanalyseur à sonde électronique (S501) ;
création de données de cartographie de la température Ms à partir des données de cartographie de la composition de base ;
binarisation des données de cartographie de la température Ms (S502) ;
calcul d'une première composante principale et d'une deuxième composante principale par analyse en composantes principales des données de cartographie de la température Ms ayant subi le traitement de binarisation (S503) ; et
évaluer les caractéristiques de variation dimensionnelle de l'acier sur la base d'une relation entre la première composante principale et la deuxième composante principale.
